Europäisches Patentamt

European Patent Office

Office européen des brevets

(19)

(11) Numéro de publication: **0 180 490**
**A1**

(12) **DEMANDE DE BREVET EUROPEEN**

(21) Numéro de dépôt: **85401772.0**

(22) Date de dépôt: **12.09.85**

(51) Int. Cl.⁴: **A 61 K 39/385,** A 61 K 39/295

(30) Priorité: **12.09.84 FR 8413989**

(43) Date de publication de la demande: **07.05.86**
**Bulletin 86/19**

(84) Etats contractants désignés: **AT BE CH DE FR GB IT LI LU NL SE**

(71) Demandeur: **AGENCE NATIONALE DE VALORISATION DE LA RECHERCHE (A.N.V.A.R.) Etablissement public de droit français, 43, rue de Caumartin, F-75436 Paris Cédex 09 (FR)**

(72) Inventeur: **Chedid, Louis, 16-18, rue Gaston Cavaillet, F-75015 Paris (FR)**
Inventeur: **Jolivet, Michel, 6, rue Edouard Branly, F-92130 Issy Les Moulineaux (FR)**
Inventeur: **Audibert, Françoise, 44, rue Ybry, F-92200 Neuilly Sur Seine (FR)**
Inventeur: **Tartar, André, Rue du Moulin, F-62490 Vitry En Artois (FR)**
Inventeur: **Gras-Masse, Hélène, 29, rue de la Rosière Merignies, F-59710 Pont a Marcq (FR)**

(74) Mandataire: **Gutmann, Ernest et al, S.C. Ernest Gutmann - Yves Plasseraud 67, boulevard Haussmann, F-75008 Paris (FR)**

(54) **Molécule synthétique contenant une pluralité d'épitopes distincts, procédé pour son obtention et application à la production de polyvaccins.**

(57) L'invention concerne une composition de polyvaccin. Elle contient une molécule synthétique caractérisée par la réunion dans une seule molécule d'une pluralité d'haptènes distincts conjugués deux à deux ou les uns à d'autres par l'intermédiaire de liaisons covalentes, ces haptènes distincts étant respectivement porteurs d'épitopes ou de sites antigéniques également distincts.

MOLECULE SYNTHETIQUE CONTENANT UNE PLURALITE D'EPITOPES DISTINCTS, PROCEDE POUR SON OBTENTION ET APPLICATION A LA PRODUCTION DE POLYVACCINS.

L'invention est relative à une molécule synthétique contenant plusieurs épitopes distincts et à l'application de cette molécule à la constitution de polyvaccins.

On sait qu'un certain nombre de vaccins synthétiques ou semi-synthétiques ont déjà été proposés ou même réalisés. D'une façon générale, il a déjà été proposé de substituer à des antigènes naturels utilisés en vaccination humaine ou vétérinaire des antigènes de synthèse possédant des éléments de structure en commun avec ces antigènes naturels ou "natifs". Ces éléments de structure contiennent en général un ou plusieurs sites antigéniques ou "épitopes" de l'antigène naturel. Par exemple cet antigène synthétique contient une séquence peptidique que l'on retrouve également dans l'antigène naturel ou "natif", lorsque celui-ci consiste en une protéine ; ou il peut consister en un oligosaccharide lorsque l'antigène naturel possède une structure polysaccharidique. Cependant, sauf exception, ces peptides ou oligosaccharides de synthèse, qui sont effectivement susceptibles d'être reconnus par des anticorps à l'égard de l'antigène naturel, ne présentent eux-mêmes, in vivo, qu'une activité immunogène extrêmement faible, voire non décelable par les techniques immunologiques courantes. En d'autres termes, ils se comportent comme des haptènes. Cette dernière expression sera effectivement utilisée dans ce qui suit pour désigner tout produit de synthèse, tel qu'il a été défini plus haut, qui en particulier comporte au moins un épitope caractéristique d'un antigène naturel.

Diverses méthodes ont été proposées dans la littérature pour renforcer cette immunogénicité.

Il a par exemple été proposé de les "oligoméri- ser". Cette dernière opération entraîne en effet dans certains cas un certain accroissement du pouvoir immunogène. Les anticorps formés ne présentent cependant pas toujours un pouvoir protecteur prononcé à l'égard des antigènes natifs correspondants.

Une autre méthode couramment étudiée consiste dans la conjugaison covalente de l'haptène ou du polymère de l'haptène avec une molécule porteuse physiologiquement acceptable et de poids moléculaire suffisamment élevé. Cette conjugaison peut effectivement conduire à un accroissement sensible de l'immunogénicité susceptible d'être induite in vivo.

Le plus souvent, les molécules porteuses sont constituées par une molécule de haut poids moléculaire, possédant elle-même une activité immunogène marquée. Il s'agit par exemple de l'anatoxine tétanique ou de l'anatoxine diphtérique. De telles molécules porteuses ne semblent pas devoir être utilisables pour des vaccinations répétées, soit qu'elles soient causes de chocs anaphylactiques, soit qu'elles entraînent une immunosuppression irréversible, comme on a pu l'observer dans certains cas.

Il a également été proposé de mettre en oeuvre des molécules porteuses entièrement synthétiques. La plupart des supports entièrement synthétiques qui ont été proposés risquent de présenter un niveau de toxicité qui les rendraient impropres à la constitution de principes actifs de vaccins. A l'heure actuelle, un seul support synthétique connu s'est montré à peu près dépourvu de toxicité. Il s'agit de la multi-DL-alanyl-poly-L-lysine (A--L) ayant un poids moléculaire de l'ordre de 80.000. Mais l'utilisation de ce support n'est vraiment efficace que si des groupes muramylpeptide sont également greffés, sur ses chaînes, par l'intermédiaire de liaisons covalentes.

A l'action immunogène alors importante susceptible d'être induite par l'haptène s'ajoute alors aussi une immunogénicité marquée à l'égard des groupes muramylpeptide et de A--L. On se trouve en définitive ramené à des difficultés semblables à celles qui sont observées avec les molécules porteuses "non synthétiques".

Plus récemment une autre approche des problèmes de la vaccination a été présentée. Dans l'article de "Biochemical and Biophysical Research Communications" (Vol. 117, No. 3, 1983, 28 décembre 1983, pages 908-915), intitulé "Antibody responses elicited by a polyvalent vaccine containing synthetic diphteric, streptococcal and hepatitis peptides coupled to the same carrier", L. CHEDID et Coll., ont proposé la constitution d'un polyvaccin porteur des trois peptides synthétiques comportant respectivement des épitopes de la toxine diphtérique, de la protéine M du streptococcus type 24 et de l'antigène de surface du virus de l'hépatite B (HBs), tous trois conjugués à une molécule porteuse constituée par l'anatoxine tétanique. Ce "polyvaccin" s'est avéré induire chez l'animal la production d'anticorps respectivement dirigés contre les différents épitopes, sans que l'on observe une domination marquée de l'un des épitopes sur les autres, au niveau des réactions immunogènes de chacun d'entre eux. Ce premier vaccin n'en présente pas moins les inconvénients susmentionnés tenant à la présence d'une molécule porteuse de haut poids moléculaire.

L'invention a pour but de remédier, au moins en grande partie, à ces difficultés, plus particulièrement de fournir des molécules entièrement synthétiques, utilisables pour la production de polyvaccins susceptibles de conférer des protections simultanées contre une pluralité d'agents pathogènes distincts, dans des conditions qui peuvent être plus aisément contrôlées au niveau de l'administration à l'hôte.

La molécule synthétique conforme à l'invention est caractérisée par la réunion dans une seule molécule d'une pluralité d'haptènes distincts conjugués deux à deux ou les uns à d'autres par l'intermédiaire de liaisons covalentes, cette molécule synthétique étant exempte de toute chaîne ou groupe porteur ayant un poids moléculaire plus élevé que celui de chacun des susdits haptènes distincts, ces haptènes distincts étant respectivement porteurs d'épitopes ou de sites antigéniques également distincts, chacun de ces haptènes correspondant à un antigène ou à une autre molécule contre laquelle une vaccination ou l'induction d'anticorps est recherchée.

La molécule synthétique ainsi obtenue présente alors - en l'absence de toute molécule porteuse de haut poids moléculaire - des propriétés immunogènes suffisantes, le cas échéant après amplification à l'aide d'un adjuvant immunologique du type muramylpeptide associé à cette molécule, pour induire in vivo des réactions immunitaires à l'égard des haptènes considérés, mais également une action protectrice à l'égard des antigènes de masses moléculaires plus importantes et porteurs des mêmes épitopes, notamment d'agents pathogènes contre lesquels une protection est recherchée.

Dans ce qui précède, on donne au terme "haptène" un sens très large. Il s'étend à tout haptène ou fragment haptènique de faible poids moléculaire, indépendamment de son mode d'obtention, que ce soit par exemple par fractionnements, dépolymérisation, synthèse ou recombinaison génétique.

Egalement dans ce qui précède, l'expression "site antigénique" ou "épitope" signifie toute configuration, notamment de surface, de l'haptène susceptible d'être reconnue par un anticorps préalablement formé à l'égard d'un antigène ayant une séquence, par exemple polypeptidique, en commun avec cet haptène. L'haptène dont la conformation peut être modifiée par la conjugaison à d'autres haptènes, conformément à l'invention, induit in vivo des anticorps ou une réaction spécifique à médiation cellulaire, non seulement contre lui-même, mais en outre - dans le cas où l'haptène a un élément de structure en commun avec un antigène natif - contre l'antigène natif lui-même ou l'antigène que l'on peut éventuellement former par couplage de l'haptène avec un support macromoléculaire ou encore avec un muramylpeptide. Par exemple, un premier haptène consistant en une séquence comprenant un nombre réduit d'acides aminés peut, quand il est couplé à un second haptène, induire _in vivo_ la formation d'anticorps actifs contre le polypeptide ou la protéine antigénique ayant en commun avec le premier haptène la même séquence d'acides aminés, la même réaction se produisant en ce qui conerne le deuxième haptène. Il en est naturellement de même en ce qui concerne des haptènes non exclusivement peptidiques. A cet égard, un ou plusieurs des haptènes consistent par exemple en une ou plusieurs structures osidiques de poids moléculaires réduits. Ils sont par exemple constitués d'un monosaccharide ou de plusieurs monosaccharides liés entre eux par exemple par une liaison de type glycosidique. Ces oligosaccharides peuvent être soit linéaires, soit ramifiés. Ils peuvent appartenir à la famille des hexoses ou des pentoses ; ils peuvent être neutres ou aminés, contenir un acide uronique ou un acide sialique. Ils peuvent porter des substituants usuellement contenus dans les structures naturelles, tels que acétyle, sulfate ou phosphate. Ces indications sont données à titre d'exemples mais ne sont pas limitatives et l'on envisage d'une manière générale toutes les molécules synthétiques résultant de la conjugaison entre eux de tous haptènes peptidiques et/ou osidiques dont on retrouve les équivalents à l'intérieur des structures des antigènes correspondants de masses moléculaires élevées.

Il est entendu que, lorsque dans la suite il sera fait référence à des "polyvaccins", il s'agit de toute molécule synthétique conforme à l'invention et susceptible d'induire la production d'anticorps distincts, reconnaissant spécifiquement les structures hapténiques ou antigéniques correspondantes. De façon générale, le langage qui précède s'étendra à toute réaction immunitaire induite, y compris les réactions à médiation cellulaire.

D'une façon générale, tout haptène ayant un élément de structure en commun avec un antigène connu à l'égard duquel une protection peut être recherchée, peut être utilisé dans la fabriction - par conugaison avec des haptènes distincts - de polyvaccins conformes à l'invention. On mentionnera, à titre d'exemple des antigènes en question, des principes actifs extraits de micro-organismes infectieux ou les micro-organismes infectieux eux-mêmes (vaccins atténués ou tués). Plus particulièrement, les haptènes susceptibles d'être mis en oeuvre dans le cadre de l'invention peuvent avoir en commun des éléments de structure avec des constituants de ces agents pathogènes (tels que bactéries, virus parasites, rickettsies, protozoaires, etc.). A titre d'exemple, on mentionnera les haptènes ayant des éléments de structure en commun avec des protéines antigéniques appartenant aux enveloppes des virus de l'hépatite virale B, de la grippe, des virus herpétiques, ou encore des protéines de parois bactériennes, par exemple streptococciques, etc.. Au titre des haptènes ayant des éléments de structure communs avec des antigènes non exclusivement peptidiques ou non peptidiques, on peut mentionner des oligosaccharides, glyco-peptides, lipopeptides, glycolipides, lipides, stéroïdes, etc.. De façon plus générale, on peut aussi se reporter aux indications fournies dans les demandes de brevets européens n° 0 003 833 et n° 0 089 290.

L'invention s'étend plus particulièrement aux polyvaccins mettant en oeuvre une pluralité d'haptènes ou de fragments hapténiques de faibles poids moléculaires qui peuvent être obtenus par synthèse chimique.

En particulier, l'invention concerne des polyvaccins mettant en jeu des haptènes distincts dont les poids moléculaires respectifs ne dépassent pas environ 5.000 et avantageusement sont inférieurs à 3.000.

Il est tout à fait remarquable que chaque haptène joue le rôle de molécule porteuse vis-à-vis des autres haptènes inclus dans le même polyvaccin. Et cela est d'autant plus remarquable que, de préférence, le polyvaccin synthétique présentera en général dans son ensemble un poids moléculaire total inférieur à celui des seules molécules porteuses utilisées jusqu'à ce jour. On mentionnera, sans que cela soit limitatif, que les polyvaccins selon l'invention présenteront en général des poids moléculaires se situant entre environ 6.000 et environ 60.000, de préférence entre environ 10.000 et environ 40.000.

Des polyvaccins préférés comprennent de 2 à 10 séquences hapténiques portant des épitopes distincts. Plus avantageusement encore, ils en comportent de 4 à 6. Il va de soi que fait également partie de l'invention tout polyvaccin dans lequel un ou plusieurs épitopes seraient présents en plusieurs exemplaires, les nombres d'exemplaires variant d'un épitope à l'autre.

Bien entendu, les polyvaccins selon l'invention peuvent comporter, outre les séquences d'haptènes choisies, des éléments de liaison chimique entre chacun des haptènes distincts. Il va naturellement de soi que ces éléments de liaison ne présentent alors que des poids moléculaires réduits les rendant sans rapport aucun avec des molécules porteuses classiques. D'une façon générale, les éléments de liaison ne contribuent que dans une proportion mineure aux poids moléculaires moyens des polyvaccins formés : leur proportion pondérale relative ne dépassera en général pas 30 % en poids du poids moléculaire total moyen du polyvaccin. Ces éléments de liaison ont des poids moléculaires respectifs qui, en général, sont inférieurs à 500, même de préférence inférieurs à 200.

Ces éléments de liaison sont en général choisis parmi des éléments inertes et dépendent du choix des réa- tifs qui sont mis en oeuvre pour réaliser la conjugaison des divers haptènes entre eux. L'invention ne saurait être limitée à aucune structure particulière du polyvaccin. A titre d'exemple, les différents haptènes entrant dans la constitution de ces polyvaccins peuvent être reliés deux à deux pour former une chaîne. Dans une autre forme de réalisation, des haptènes distincts peuvent être greffés sur un haptène différent commun, notamment lorsque celui-ci comprend en divers points de sa chaîne, des groupements fonctionnels libres, susceptibles d'être mis en jeu dans les réactions de conjugaison avec les groupements fonctionnels portés par d'autres haptènes. Par exemple, lorsque les différents haptènes sont de natures peptidiques, les groupes fonctionnels du genre en question sont constitués par des groupes $-NH_2$, $-COOH-$, SH, etc.. Par exemple, le peptide SCB7 dont question plus loin peut jouer ce rôle d'haptène porteur à l'égard d'autres haptènes, ne serait-ce que parce qu'il porte lui-même 6 groupes $-NH_2$ susceptibles d'être mis en oeuvre dans des réactions de conjugaison avec d'autres haptènes.

Il va de soi aussi que dans ce qui précède, tout haptène entrant dans la constitution du polyvaccin selon l'invention peut lui-même être constitué par un oligomère formé à partir de plusieurs motifs monomères qui répondent eux-mêmes à la définition qui a été donnée plus haut de l'haptène. Par exemple, cet oligomère peut contenir de 2 à 10 de ces motifs.

L'invention concerne aussi un procédé pour fabriquer des polyvaccins synthétiques conformes à l'invention, ce procédé consistant à faire réagir un premier haptène comprenant une première fonction réactive avec un second haptène (ou plusieurs haptènes) pourvu d'une seconde fonction réactive (ou de plusieurs fonctions réactives) complémentaires de celle du premier haptène, les fonctions réactives distinctes éventuellement présentes sur certains de ces haptènes et n'intervenant pas dans la réaction (ou les réactions) étant si besoin protégées. Il est entendu que dans ce qui précède "complémentarité" signifie la capacité des fonctions réactives de deux haptènes distincts de conduire à une réaction de couplage covalent, directe ou indirecte, si besoin en présence d'un agent également bifonctionnel ou simplement d'un agent chimique favorisant la réaction de couplage covalent.

De préférence, le premier haptène comprend au moins une fonction amine, carboxyle libre, ester ou alcool susceptible d'être mise en jeu dans la réaction de couplage avec une fonction correspondante portée par le second haptène.

Conformément à une première variante du procédé selon l'invention, on réalise la condensation entre un premier haptène portant au moins une fonction amine libre et un second haptène portant également au moins une fonction amine libre et l'on procède à la condensation de ces deux haptènes, en présence d'un réactif difonctionnel tel que, par exemple, la glutaraldéhyde ou la benzoquinone.

Selon une autre variante du procédé de l'invention, on fait réagir le premier haptène portant une fonction réactive amine, carboxyle, hydroxyle ou sulfhydryle selon l'une des techniques couramment utilisées en synthèse peptidique, avec le second haptène qui porte alors lui-même une fonction réactive complémentaire amine, carboxyle, hydroxyle, alcool ou sulfhydryle.

Lorsque l'on réalise le couplage entre une fonction carboxyle portée par l'un des partenaires avec une fonction amine portée par l'autre partenaire de la réaction, on réalise avantageusement la réaction en phase aqueuse en présence d'un carbodiimide hydrosoluble ou d'un carbodiimide liposoluble au sein d'un solvant organique inerte, tel que le diméthyl-formamide (DMF) ou le tétrahydrofuranne (THF), acétate d'éthyle, chlorure de méthylène, etc..

Des carbodiimides hydrosolubles avantageux sont constitués par le N-cyclohexyl-N'(bêta-(N-méthyl-morpholino)-éthyl)-carbodiimide p-toluène sulfonate, le chlorhydrate de (3-méthyl)-aminopropyl-carbodiimide.

Des carbodiimides appropriés pour des réactions au sein d'un solvant organique sont par exemple constitués par le N,N'-dicyclohexyl-carbodiimide.

Selon une autre variante du procédé selon l'invention, on fait réagir la fonction carboxyle portée par l'un des partenaires avec un chlorocarbonate d'alkyle ($C_2$ à $C_4$), puis l'on fait agir l'anhydride mixte obtenu à partir de ce premier partenaire avec le second partenaire portant la fonction amine devant intervenir dans le couplage au sein d'un solvant organique inerte, tel que ci-dessus défini, ou d'une phase aqueuse.

La première étape de la réaction est effectuée par exemple dans le diméthylformamide, en présence d'une amine tertiaire, par exemple la N-méthyl-morpholine, en utilisant par exemple le chlorocarbonate d'isobutyle. Après trois minutes à -15°C, on ajoute au mélange réactionnel le partenaire porteur d'une fonction amine déprotonée, par exemple avec de la N-méthyl-morpholine.

Selon une troisième variante du procédé selon l'invention, on fait réagir un ester activé préalablement formé de celui des partenaires portant le groupe carboxylique devant intervenir dans la réaction de couplage, avec l'autre partenaire portant une fonction amine. Des esters activés avantageux sont l'ester p-nitrophénylique ou l'ester N-hydroxysuccinimidique.

Ces réactions sont conduites dans un solvant organique inerte, tel que le diméthyl-formamide.

Selon une autre variante encore du procédé selon l'invention, on fait réagir l'un des partenaires portant une fonction sulfhydryle avec l'autre partenaire, lequel porte alors un groupe maléimide.

On peut encore faire réagir avec le partenaire portant le groupe sulfhydryle l'autre partenaire portant un groupe 6-maléimido-caproïque-acide ou un groupe 3-(2-pyridyl-dithio)propionate.

Les fonctions réactives ne devant pas intervenir dans la réaction peuvent être protégées par des groupes de blocage classiques.

Les groupes carboxyliques peuvent notamment être protégés par des groupes benzylique, benzylidène ou anizilidène.

Ces groupes esters peuvent ensuite être éliminés notamment par hydrogénolyse. Celle-ci permet d'ailleurs également l'élimination simultanée d'autres groupes protecteurs tels que les groupes N-carbobenzoxy.

Les fonctions amine sont avantageusement protégées par des groupes benzyl-oxycarbonyle, t-butyl-oxycarbonyle ou toluène sulfonyle. Ces groupes peuvent ensuite être éliminés par hydrogénation catalytique ménagée, par exemple en présence de palladium, ou par acidolyse ou encore par action du sodium dans l'ammoniac liquide.

Les fonctions sulfhydryle n'intervenant pas dans la réaction de couplage peuvent être protégées par des groupes acétamidométhyle ou formamidométhyle.

Bien entendu, on peut avoir recours à tout autre type de liaison, mettant en jeu les combinaisons pouvant intervenir entre une fonction sulfhydrile portée par l'un des partenaires et un groupe maléimide porté par l'autre partenaire, par exemple en faisant usage de la technique décrite par T. KITAGAWA et T. AIKAGAWA dans "J. Biochem." 79 (1976), 233.

La conjugaison peut également se faire en utilisant les modes classiques de diazotisation ou de réaction mettant en jeu un isothiocyanate, notamment lorsque l'un des haptènes porte une fonction aromatique aminée, et lorsque l'autre haptène porte une fonction amine susceptible d'intervenir dans cette réaction. De telles techniques de réalisation sont décrites par exemple par B.F. ERLANGER dans "Pharm- col. Rev.", 25 51973, p. 271.

Le couplage peut également être réalisé par la réduction d'une base de Schiff réalisée entre une fonction aldéhyde portée par l'un des haptènes et une fonction amine portée par l'autre haptène (par exemple selon la technique de G.R. GRAY, "Arch. Biochem. Biophys.", 163 (1974), p. 425).

Toutes ces réactions sont en soi bien connues et le spécialiste appréciera que de nombreuses variantes peuvent être aménagées pour aboutir aux mêmes résultats. On remarquera aussi que les conjugaisons entre haptènes peuvent s'effectuer au moyen d'un pontage, l'agent de pontage consistant par exemple en un réactif bifonctionnel. En l'occurrence, le groupe de pontage entre deux haptènes dans le conjugué final de préférence n'excèdera pas la longueur d'un enchaînement correspondant à celle d'une chaîne hydrocarbonée p-décylique.

De tels agents de pontage sont par exemple mentionnés dans le brevet France n° 78 16792 déposé le 5 juin 1979.

Dans ce qui précède, il a surtout été évoqué le cas du couplage entre des haptènes de natures peptidiques. Lorsque les haptènes sont constitués par des oligosaccharides, les mêmes types de réactions peuvent être mis en oeuvre, dès lors que l'un des haptènes possède une fonction carboxylique libre ou une fonction amine, ou encore une fonction alcool pouvant alors former des liaisons amide ou ester avec des fonctions adéquates portées par l'autre haptène.

Dans toutes les réactions qui précèdent, les proportions relatives des haptènes mis en jeu peuvent être variées selon les proportions finales désirées de chaque haptène dans le polyvaccin final. En particulier, ces proportions relatives seront ajustées en fonction du nombre de groupes fonctionnels portés par chacun d'eux et susceptibles d'entrer dans la réaction de conjugaison avec des groupes fonctionnels complémentaires portés par d'autres haptènes.

Des caractéristiques supplémentaires de l'invention apparaîtront encore au cours de la description qui suit d'exemples de production de polyvaccins synthétiques et de leurs propriétés immunogènes.

EXEMPLE I : Production d'un polyvaccin mettant en jeu quatre haptènes distincts.

Les haptènes mis en jeu ont été les suivants :

1°) SODP : octodécapeptide contenant une séquence de la "boucle diphtérique" et de formule :

$H_2N$-Ala-Ala-Cys-Ala-Gly-Asn-Arg-Val-Arg-
Arg-Ser-Val-Gly-Ser-Ser-Leu-Lys-Cys.

2°) SCB7 : peptide contenant un épitope caractéristique du streptococcus type M24 contenant la séquence :

Asn-Phe-Ser-Thr-Ala-Asp-Ser-Ala-Lys-
Ile-Lys-Thr-Leu-Glu-AlaGlu-Lys-Ala-Ala-
Leu-Ala-Ala-Arg-Lys-Ala-Asp-Leu-Glu-Lys-
Ala-Leu-Glu-Gly-Ala

3°) HBs (peptide 99-121) : peptide comprenant un site antigénique de l'enveloppe du virus contenant la séquence :

Asp-Tyr-Gln-Gly-Met-Leu-Pro-Val-Cys-Pro-Leu-Ile-
     100                                  110
Pro-Gly-Ser-Ser-Thr-Thr-Ser-Thr-Gly-Pro-Cys.
                                 120

4°) DDP Mal : peptide portant un épitope caractéristique d'un antigène protecteur contre Plasmodium Knowlesi, agent de la malaria du singe, décrit par GODSON G.N. et Coll. (1983), Nature, 305, 29-33, constitué par la séquence :

Tyr-Gln-Ala-Gln-Gly-Asp-Gly-Ala-Asn-Ala-
Gly-Gln-Pro-Gln-Ala-Gln-Gly-Asp-Gly-Ala-Asn-
Ala-Gly-Gln-Pro-Cys

1°) <u>Préparation du polyvaccin</u> :

5 équivalents NH$_2$, soit 12,5 mg de HBs 99-121, 5 équivalents NH$_2$, soit 14,5 mg de DDP Mal, 10 équivalents NH$_2$, soit 9 mg de SODP et 20 équivalents NH$_2$, soit 26 mg de SCB7, sont dissous dans une solution de bicarbonate de sodium 0,1 M (10 ml), mélangée avec une solution aqueuse de 4 microlitres de glutaraldéhyde à 25 % pour obtenir une concentration finale de 0,1 % en glutaraldéhyde. La réaction s'effectue à température ambiante, à l'obscurité et sous agitation. On laisse la réaction se poursuivre pendant 3 jours. La solution est ensuite dialysée contre un tampon approprié, tel que le PBS, dans des conditions permettant de séparer tous les constituants ayant des poids moléculaires inférieurs à 12.000 daltons. La solution retenant les poids moléculaires plus élevés est ensuite soumise à une opération de chromatographie liquide, sous haute pression, dans une colonne LK13 selon les prescriptions du fabricant. La lecture a été faite à 295 nm.

Le diagramme d'élution fait apparaître un pic unique homogène qui a été recueilli en six fractions d'élution successives. Les poids moléculaires moyens de ces fractions s'échelonnent entre 12 000 et 40 000. L'analyse immunochimique du contenu de chacune de ces fractions fait apparaître la présence de molécules contenant des séquences peptidiques correspondant à chacun des haptènes initialement mis en jeu. Le rendement de conjugaison a été de 95 %.

Le contenu de ces fractions a été réuni et la solution obtenue lyophilisée.

2°) <u>Propriétés biologiques du polyvaccin</u> :

a) <u>Induction d'anticorps in vivo.</u>

Des souris Swiss ont été réparties en trois groupes. Ces groupes ont reçu par voie sous-cutanée 100 microgrammes du polyvaccin avec respectivement :

- de l'eau physiologique (groupe <u>a</u>),

- de l'adjuvant complet de Freund (ACF) (groupe b),
-  100 microgrammes de l'alpha-butyl-ester de la N-acétyl-muramyl-L-alanyl-D-glutamine (murabutide) (groupe c).

Un  mois plus tard, les souris reçoivent toutes un rappel  de  polyvaccin (100 microgrammes par souris), puis au  70ème jour après la première injection, un second rappel de 100 microgrammes du polyvaccin.

Au jour 78, les animaux sont saignés et leurs taux d'anticorps  sont titrés contre chacun des quatre peptides dans  des systèmes ELISA, dans des conditions identiques à celles  décrites  dans l'article mentionné plus haut de L. CHEDID et al.

Les résultats figurent dans le tableau 1 ci-après. Ils  sont  fournis  souris  par souris. Dans la colonne de gauche  sont  indiqués  les constituants utilisés pour les injections  primaires.  Dans les quatre colonnes de droite figurent  les  réponses  individuelles  enregistrées  pour chaque  souris  à  l'égard des quatre haptènes individuels définis plus haut.

## TABLEAU 1

| Injection primaire | N° souris | Réponses individuelles anti : | | | |
|---|---|---|---|---|---|
| | | SODP | SCB7 | HBs(99-121) | DDP Mal |
| Témoin | 1 | 250 | 150 | 150 | <100 |
| polyvaccin | 2 | <100 | <100 | <100 | <100 |
| en eau | 3 | <100 | <100 | <100 | <100 |
| physiolo- | 4 | <100 | <100 | <100 | <100 |
| gique | 5 | <100 | <100 | <100 | <100 |
| | 6 | <100 | <100 | <100 | <100 |
| | 7 | <100 | <100 | <100 | <100 |
| | 8 | <100 | <100 | <100 | <100 |
| Polyvaccin | 9 | 52 000 | 140 000 | 110 000 | 290 000 |
| + | 10 | 93 000 | 60 000 | 68 000 | 220 000 |
| ACF | 11 | 15 000 | 305 000 | 140 000 | 300 000 |
| | 12 | 7 500 | 80 000 | 100 000 | 250 000 |
| Polyvaccin | 13 | 105 000 | 100 000 | 80 000 | 105 000 |
| + | 14 | 130 000 | 14 000 | 15 000 | 110 000 |
| murabutide | 15 | 6 200 | 400 000 | 1 700 | 5 000 |
| 100 $\mu$g | 16 | 7 500 | 8 000 | 1 740 | 3 500 |

Les résultats permettent de faire les observations suivantes :

- tous les haptènes s'expriment immunologiquement, même si les niveaux de réponse observés au niveau des différentes souris ne sont pas toujours semblables ; on remarquera néanmoins que dans l'ensemble les niveaux d'immunogénicité sont élevés ;

- il n'y a pas de compétition antigénique entre les quatre constituants du polyvaccin.

b) <u>Mise en évidence d'une immunité protectrice</u>
<u>contre le streptococcus pyogenes type M24</u> :

Un certain nombre de souris ont été immunisées dans les mêmes conditions que les souris du groupe <u>c</u> (deux rappels aux moments indiqués ci-dessus avec la combinaison polyvaccin + murabutide). Elles ont été éprouvées au jour 78 par une dose, mortelle pour des témoins, de <u>streptococ-</u><u>cus</u> <u>pyogenes</u> de type M24. Une proportion substantielle des souris ainsi éprouvées ont survécu. Cet essai témoigne que l'immunisation préalable des souris éprouvées s'est manifestée par la production d'anticorps capables de reconnaître la structure naturelle de l'agent pathogène. Au surplus, la survie d'un certain nombre des animaux a manifesté l'activité biologique de protection des anticorps induits par le polyvaccin.

L'exposé des résultats des essais qui suivent démontrent l'activité protectrice notable observée contre au moins deux des agents pathogènes concernés par le polyvaccin.

1°) <u>Activité antistreptocoques</u>

Les souris ont reçu 100 µg du vaccin tétravalent, trois fois à un mois d'intervalle. Un groupe a reçu le vaccin seul, le second le vaccin avec du FCA (adjuvant complet de Freund) et le troisième avec du murabutide. A la fin de l'expérience, les souris ont reçu par la voie péritonéale une injection d'épreuve de 100.000 germes pyogènes souche 24. Les résultats sont donnés sur le

0180490

18

tableau 2 où figurent les titres individuels antipeptides S-CB7 et anti-M24 mesurés sur les sérums prélevés deux jours avant l'épreuve. Les animaux n'ayant pas survécu sont indiqués par des croix. Les chiffres entre parenthèses donnent les jours où les souris en cause sont mortes. Un lot vacciné comprenant 8 souris a reçu la même dose de germes$^+$ et il n'y a eu aucun survivant. Il faut noter que le lot traité par le murabutide est aussi bien protégé que le lot FCA.

2°) Activité anti-Plasmodium knowlesi

L'activité biologique des sérums prélevés chez des souris et des cobayes préalablement immunisés ont été testés par leur capacité à provoquer la réaction dite CSP de l'anglais ("circumsporozoïte protein precipitation") ou précipitation des protéines circumsporozoïtes sur des sporozoïtes vivants, selon la technique décrite par Nordin et Coll., 1979. Des résultats typiques figurent dans le tableau 3. Les chiffres de la colonne CSP indiquent le nombre de sporozoïtes ayant montré une réaction de précipitation sur le nombre total de sporozoïtes examinés. Le score indique l'intensité de cette réaction. Les sérums étaient testés au 1/10°.

TABLEAU 2

Protection contre des homologues streptocoques

| Souris n° | Vaccin tétravalent en solution saline | | | Vaccin tétravalent + ACF | | | Vaccin tétravalent + Murabutide | | |
|---|---|---|---|---|---|---|---|---|---|
| | Anti-S-CB7 | Anti-M24 | mort/total (jour) | Anti-S-CB7 | Anti-M24 | mort/total (jour) | Anti-S-CB7 | Anti-M24 | mort/total (jour) |
| 1 | < 100 | < 100+ | | 60.000 | 5.400+ | | 1.900 | 2.500+ | |
| 2 | < 100 | < 100+ | 4/4 | 80.000 | 11.000 | 3/8 | 2.000 | 4.800 | 2/8 |
| 3 | 150 | 150 + | (1,2,3,5) | 98.000 | 25.000+ | (3,6,7) | 2.800 | 2.200+ | (3,5) |
| 4 | 150 | < 100+ | | 140.000 | 27.000 | | 8.000 | 3.000 | |
| 5 | | | | 144.000 | 48.000+ | | 10.700 | 8.000 | |
| 6 | | | | 305.000 | 61.000 | | 14.000 | 6.000 | |
| 7 | | | | 345.000 | 63.000 | | 100.000 | 12.000 | |
| 8 | | | | 390.000 | 92.000 | | 400.000 | 20.000 | |

19

0180490

TABLEAU 3

Activité antisporozoïte mesurée par la réaction CSP sur des sérums de cobaye et de souris après immunisation avec le vaccin tétravalent

| Traitement | Animaux | Anti-DDP-Mal | CSP | |
|---|---|---|---|---|
| | | titres | Nb. de CSP$^+$ | Score |
| Vaccin tétravalent | souris | < 100 | – | – |
| Vaccin tétravalent + ACF | souris " | 275.000 60.000 | 16/25 9/25 | ++ ++++ ± |
| Vaccin tétravalent + murabutide | souris " | 110.000 5.000 | 11/25 – | ++ – |
| Vaccin tétravalent | cobayes | 27.000 | 17/25 | ++ |
| Vaccin tétravalent + ACF | " | 64.000 | 18/25 | ++ ++++ |
| Vaccin tétravalent + murabutide | " | 12.000 | 18/25 | ++ +++ |

EXEMPLE II :

D'autres polyvaccins peuvent être constitués de la même manière, par exemple en groupant des séquences portant des épitopes obtenus à partir des antigènes identifiés ci-après ou correspondant aux maladies ci-après. Il s'agit par exemple des 4 polyvaccins suivants qui regroupent respectivement des haptènes portant des épitopes correspondant à :

     a) choléra-poliomyélite-influenza,

     b) Plasmodium falciparum-HBs,

     c) colibacille-choléra-rotavirus,

     d) LHRH-fièvre aphteuse-leucose bovine.

EXEMPLE III :

D'autres polyvaccins peuvent être orientés plus généralement vers le même type d'affections, surtout lorsque celles-ci peuvent être induites par des antigènes de sérotypes différents ; par exemple les divers types de virus de la poliomyélite ou de la grippe.

En particulier l'invention concerne aussi une molécule synthétique caractérisée par le fait que les divers haptènes distincts qu'elle contient sont originaires de la même catégorie d'antigène.

En particulier l'invention concerne un polyvaccin contenant une pluralité de séquences porteuses protectrices contre le virus de la poliomyélite, ces différentes séquences correspondant notamment à des épitopes normalement contenus dans les protéines VP1, VP2 et VP3 appartenant à un poliovirus du même type et notamment du type 1. ainsi l'invention concerne un polypeptide contenant au moins deux haptènes liés bout à bout (ou par l'intermédiaire de courts peptides distincts) ayant des séquences polypeptidiques en commun :

- soit avec les séquences protéiques VP1, VP2, VP3 ou VP4 d'un poliovirus de même type,

- soit avec les mêmes types de protéines, par exemple les séquences protéiques VP1 ou VP2, issues de poliovirus de types différents (notamment types 1, 2 et/ou 3),

- soit encore avec des séquences protéiques de types différents, elles-mêmes originaires de poliovirus de types différents, par exemple avec une séquence protéique VP2 issue d'un poliovirus du type 1 et une séquence protéique VP1 issue d'un poliovirus de type 3.

Ces séquences peuvent être liées l'une à l'autre dans un ordre quelconque, par des techniques bien connues de l'homme du métier dans la chimie des protéines. Par exemple les séquences sont liées deux à deux en mettant chaque fois à profit la fonction C-terminale de l'un des polypeptides, et l'extrémité N-terminale de l'autre polypeptide, dans une réaction de conjugaison en présence de carbodiimide, l'opération étant répétée autant de fois que l'on veut introduire de groupements peptidiques dans la molécule synthétique finale.

Il va sans dire que les fonctions réactives, ne devant pas entrer dans la réaction, doivent être convenablement protégées.

A titre d'exemples, on peut fabriquer des molécules synthétiques contenant au moins deux haptènes distincts choisis parmi ceux qui suivent, soit à l'intérieur de l'un des groupes A), B), C) et D) qui suivent, soit au sein de plusieurs d'entre eux :

Groupe A : peptides de la séquence protéque VP1 du polyovirus de type 1.

- 93 → 103 Asp-Asn-Pro-Ala-Ser-Thr-Thr-Asn-Lys-Asp-Lys
  (réf. : E.A. Emini, B.E. Jameson, E. Wimmer, Nature, 1983, n° 304, p. 699-703)
- 61 → 80 Val-Gln-Thr-Arg-His-Val-Val-Gln-His-Arg-Ser-Arg-Ser-Glu-Ser-Ser-Ile-Glu-Ser-Phe
- 91 → 109 Thr-Val-Asp-Asn-Pro-Ala-Ser-Thr-Thr-Asn-Lys-Asp-Lys-Leu-Phe-Ala-Val-Trp-Lys
- 100 → 109 Asn-Lys-Asp-Lys-Leu-Phe-Ala-Val-Trp-Lys
- 182 → 201 Ser-Ile-Phe-Tyr-Thr-Tyr-Gly-Thr-Ala-Pro-Ala-Arg-Ile-Ser-Val-Pro-Tyr-Val-Gly-Ile - 222 → 241 Ala-Ala-Leu--Gly-Asp-Ser-Leu-Tyr-Gly-Ala-Ala-Ser-Leu-Asn-Asp-Phe-Gly-Ile-Leu-Ala

- et les peptides 72-104, ou mieux encore les peptides 90-104 décrits par S. Van der Werf et al (1983), Embo. J. 2-2019.

Les cinq premiers peptides ont été décrits par M. Chow, R. Yabrov, J. Bittle, J. Hogle et D. Baltimore, Proc. Natl. Acad. Sci. USA, vol. 82, p. 910-914, février 1985, Microbiologie.

Groupe B : peptides immunogènes synthétiques ayant des séquences en commun avec la séquence protéique VP2 du virus de la polio - type 1 (les numérotations utilisées ci-après se rapportent à la séquence d'ADN) publiée par Kitamura et Coll. (Nature, 1981, 291, p. 547). Il existe une autre séquence publiée par Racemello et al (PNAS, 81) qui a été utilisée par Emini).

Peptides synthétisés ou synthétisables :

VP2 (38-61) :

Trp-Pro-Glu-Tyr-Leu-Arg-Asp-Ser-Glu-Ala-Asn-Pro-Val-Asp-Gln-Pro-Thr-Glu-Pro-Asp-Val-Ala-Ala-Cys.

VP2 (72-88) :

Thr-Lys-Glu-Ser-Arg-Gly-Trp-Trp-Trp-Lys-Leu-Pro-Asp-Ala-Leu-Arg-Asp.

VP2 (162-174) :

Thr-Pro-Asp-Asn-Asn-Gln-Thr-Ser-Pro-Ala-Arg-Ser-Ser (ou un peptide voisin dans lequel le Ser terminal est remplacé par un Arg).

VP2 (145-158) :

Tyr-Gln-Asn-Ala-Asn-Pro-Gly-Glu-Lys-Gly-Gly-Thr-Phe-Thr.

VP2 (232-247) :

Pro-Leu-Ala-Pro-Leu-Asn-Phe-Val-Ser-Glu-Ser-Ser-Pro-Glu-Ile-Pro.

0180490

24

Groupe C : haptènes ayant des séquences en commun avec la séquence protéique VP2 de la protéine du virus de sérotype 3 (dont la structure primaire totale de cette protéine du polyovirus de sérotype 3 a été donnée par Glyn Stanway et al, Proc. Natl. Acad. Sc., USA, Mars 1984, vol. 81, p. 1539-1543) :

- 38-61    Trp-Pro-Glu-Phe-Ile-Arg-Asp-Asp-Glu-Ala-Asn-
           Pro-Val-Asp-Gln-Pro-Thr-Glu-Pro-Asp-Val-Ala-
           Thr-Cys.

- 71-88    Trp-Gly-Lys-Glu-Ser-Lys-Gly-Trp-Trp-Trp-Lys-
           Leu-Pro-Asp-Ala-Leu-Arg-Asp.


- 133-156  Ala-Gly-Asp-Ser-Asp-Lys-Gln-Arg-Tyr-Thr-Ser-
           Tyr-Ala-Asn-Ala-Asn-Pro-Gly-Glu-Arg-Gly-Gly-Lys-
           Phe.

- 157-174  Tyr-Ser-Gln-Phe-Asn-Lys-Asp-Asn-Ala-Val-Thr-Ser-
           Pro-Lys-Arg-Glu-Phe-Cys.

Groupe D : haptène formé à partir de la séquence 96-106 de la protéine VP1 du poliovirus de type 3 (décrit par Minor et al, Nature (1983), 301-459).

L'invention concerne également plus particulièrement ceux des peptides qui précèdent et qui sont nouveaux en eux-mêmes, ainsi que les peptides qui se distinguent des précédents par un ou plusieurs aminoacides sans cependant s'en différencier.

Dans ce qui suit on décrit les synthèses généralement applicables à la production des haptènes en cause.

Les synthèses sont réalisées selon la technique en phase solide de Merrifield.

La chaîne peptidique est préparée à partir du premier acide aminé C-terminal, fixé de manière covalente sur un polymère de styrène et divinylbenzène (pouvant également être de type polyamide) par une liaison de type ester benzylique (résine chlorométhylée).

Les acides aminés suivants, vers la partie N-terminale sont ajoutés successivement par répétition du cycle d'opérations figurant au tableau 4. Les étapes principales en sont :

1) Une déprotection du groupe Boc (tertiobutyl-oxycarbonyle, utilisé pour protéger les fonctions α-aminées) au moyen d'acide trifluoroacétique (TFA) en solution dans $CH_2Cl_2$ (40 % V/V).

2) Une neutralisation des fonctions alpha-aminées au moyen de diisopropylamine (D.I.E.A.) en solution dans $CH_2Cl_2$ (5 % V/V).

3) un couplage réalisé par activation des fonctions carboxyliques des acides aminés à introduire au moyen de dicyclohexyl-carbodiimide ou en préparant des esters activés (d'orthonitrophenol, par exemple) dans le cas de Gln et Asn. Les différents réactifs de couplage sont ajoutés en excès (3 à 6 fois) par rapport à la charge de résine.

En fin de couplage, on vérifie l'absence de fonction amine libre sur la résine au moyen du test à la ninhydrine. En cas de réaction positive, le couplage est répété.

Au cours de la synthèse des fonctions réactives portées par les chaînes latérales des aminoacides sont protégées au moyen des groupes figurant au tableau 5.

En fin de synthèse, le peptide est libéré de la résine et de ses groupes protecteurs par traitement durant 1 heure à 0° C au moyen d'acide fluorhydrique anhydre contenant 10 % d'anisole ou de crésol et 10 % de diméthylsulfure (V/V).

Les peptides sont alors purifiés par gel-filtration puis chromatographie de partition en phase inversée.

L'identité est contrôlée par analyse d'acides aminés après hydrolyse acide et l'homogénéité par chromatographie sur couche mince de silice dans trois systèmes solvants différents ainsi que par chromatographie liquide haute pression en phase inverse.

TABLEAU 4

Protocole de synthèse

| Réactifs | Volume pour 1 kg de résine | Nombre d'opérations | Temps |
|---|---|---|---|
| $CH_2Cl_2$ | 15 ml | x 3 | 2 minutes |
| TFA-$CH_2Cl_2$ (2/3) | 13 ml | x 1 | 1 minute |
| TFA-$CH_2Cl_2$ (2/3) | 13 ml | x 1 | 30 minutes |
| $CH_2Cl_2$ | 13 ml | x 5 | 2 minutes |
| DIEA-$CH_2Cl_2$ (1/20) | 15 ml | x 3 | 2 minutes |
| $CH_2Cl_2$ | 15 ml | x 5 | 2 minutes |
| couplage | 15 ml | x 1 | 90 minutes |
| $CH_2Cl_2$ | 15 ml | x 5 | 2 minutes |

## TABLEAU 5

Groupes protecteurs des chaînes latérales

| Acide aminé | Groupe protecteur |
|---|---|
| Ser | éther benzylique |
| Thr | éther benzylique |
| Asp | ester benzylique |
| Lys | benzyloxycarbonyle (Z) |
| Glu | ester benzylique |
| Arg | Tosyle |
| His | Tosyle ou DNP |

Les peptides 38-61, 72-88 et 162-174, qui ont des séquences en commun avec la séquence protéique VP2 du poliovirus de type 1 sont utilisés avec un avantage particulier dans la fabrication de molécules synthétiques dans les conditions qui ont été évoquées plus haut. Ils ont montré une immunogénicité particulièrement marquée, comme en témoigne la capacité des anticorps induits par eux in vivo de reconnaître tant la séquence protéique VP2 que le virus entier dans des essais de titrage du type ELISA.

En l'occurrence les anticorps avaient été produits par immunisation de souris Swiss (trois injections de 50 microgrammes à 30 jours d'intervalles) avec lesdits peptides, préalablement fixés sur la toxine tétanique.

Dans le tableau 6 ci-après, on rapporte les résultats obtenus avec

(1) le peptide 38-61,

(2) ce peptide et ACF.

### TABLEAU 6

Réponses individuelles

| | Anti-Pep | | | Anti-virus | | |
|---|---|---|---|---|---|---|
| | 1[*] | 2[*] | 3[*] | 1 | 2 | 3 |
| 1 - 38-61 | 400 | 25.000 | 6.400 | 200 | 800 | 6.400 |
| 2 - 38-61-TT+ACF | 6400 | >25.000 | >25.000 | 1600 | 6.400 | >6.400 |

1[*] = réponse primaire

2[*] = réponse secondaire

3[*] = réponse tertiaire

On obtient respectivement des valeurs de 200, 800 et 800 avec le peptide 72-88 et 800, 6400 et 1600 avec ce même peptide et ACF dans les réponses 1, 2 et 3 anti-pep. La réponse tertiaire anti-virus fournit des valeurs de 400 et 200 respectivement avec le peptide 72-88 et 72-88+ACF et de 1600 avec le peptide 162-174 avec ou sans ACF.

L'invention concerne enfin également les compositions préparées sous forme de vaccins contenant des doses efficaces des molécules selon l'invention, en association avec un véhicule approprié. Par exemple pour un polyvaccin du type de celui de l'exemple I, une dose unitaire comporte de 0,1 à 1 mg du polyvaccin tel que décrit.

Des compositions pharmaceutiques avantageuses sont constituées par des solutions, suspensions ou liposomes injectables, contenant une dose efficace d'au moins un polyvaccin selon l'invention. De préférence, ces formes injectables sont dans une phase aqueuse stérilisée isotonique, de préférence saline ou glucosée.

— L'invention concerne plus particulièrement de telles suspensions, solutions ou formes liposomes qui sont aptes à être administrées par injections intradermiques, intramusculaires ou sous-cutanées, ou encore par scarifications.

Elle concerne également des compositions pharmaceutiques administrables par d'autres voies, notamment par voie orale ou rectale, ou encore sous forme d'aérosols destinés à venir en contact avec des muqueuses, notamment les muqueuses oculaires, nasales, pulmonaires ou vaginales.

En conséquence, elle concerne des compositions pharmaceutiques dans lesquelles un polyvaccin selon l'invention se trouve associé à des excipients pharmaceutiquement acceptables, solides ou liquides, adaptés à la constitution de formes orale, oculaire ou nasale, ou avec des excipients adaptés à la constitution de formes d'administration rectales, ou encore avec des excipients gélatineux pour l'administration vaginale. Elle concerne aussi des compositions liquides isotoniques contenant un polyvaccin selon l'invention, adaptées à l'administration sur les muqueuses, notamment oculaire ou nasale. Elle concerne enfin des compositions destinées à être utilisées dans des appareils pour administration par aérosol.

Avantageusement, les compositions vaccinales selon l'invention contiennent en outre un véhicule, tel que la polyvinyl-pyrrolidone, facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthylcellulose, les hydroxydes et phosphates d'aluminium ou tous autres adjuvants de ce type, bien connus de l'homme de l'art. On peut également - surtout pour l'application vétérinaire - utiliser également des adjuvants huileux, de préférence métabolisables comme le squalane, le squalène et des huiles végétales et même des huiles minérales.

Les compositions pharmaceutiques selon l'invention sont donc essentiellement destinées à induire chez l'hôte des réactions immunitaires se manifestant par des productions simultanées d'anticorps distincts susceptibles de le protéger simultanément contre plusieurs antigènes distincts.

Avantageusement, ces compositions pharmaceutiques contiennent en outre un muramylpeptide jouant le rôle d'adjuvant immunologique. Un muramylpeptide particulièrement préféré est constitué par le murabutide. La littérature technique concernant les muramylpeptides est cependant suffisamment développée pour qu'il soit inutile de souligner que l'homme de métier serait à même de sélectionner d'autres muramylpeptides susceptibles de produire des effets semblables au sein des compositions polyvaccinantes conformes à la présente invention.

Un intérêt majeur des associations de polyvaccins avec un muramylpeptide réside dans la possibilité de contrôler l'immunogénicité des compositions de vaccins. Il est observé que le caractère protecteur du polyvaccin peut être élevé, même lorsque les titres en anticorps susceptibles d'être induits in vivo contre les différentes séquences hapténiques contenues dans le polyvaccin ne sont pas très élevés.

Un autre avantage de la composition de vaccins selon l'invention, même lorsque celle-ci comprend également un muramylpeptide associé avec le polyvaccin (l'expression "associé" impliquant l'absence de conjugaison covalente entre le polyvaccin et le muramylpeptide), est l'absence de risque de choc anaphylactique chez le sujet la recevant, même lorsque ce sujet a auparavant déjà été vacciné soit avec un vaccin classique, soit avec un autre polyvaccin, ce vaccin classique ou cet autre polyvaccin ayant en commun une ou plusieurs séquences hapténiques avec ladite composition.

Ceci étant, il convient de mentionner que font également partie de l'invention les polyvaccins sur lesquels pourraient être greffées une ou plusieurs molécules de muramylpeptide, notamment dans les conditions décrites dans la demande de brevet européen n° 83 400525. Le muramylpeptide peut d'ailleurs lui-même constituer un groupe pontant entre deux haptènes distincts, notamment par l'intermédiaire d'une fonction carboxyle ou amine portée par son groupe glutaminyle, d'une part, et par l'intermédiaire d'une liaison au niveau de la position 6 sur son groupe saccharidique, d'autre part.

Font encore partie de l'invention, les polyvaccins dans lesquels certains des haptènes seraient constitués d'un peptide naturel ou d'un fragment de peptide naturel, tel qu'une hormone, dont on veut pallier les effets. Ces derniers types de compositions pharmaceutiques peuvent être particulièrement utiles en thérapeutique humaine ou vétérinaire, chaque fois que sera recherchée une modification de l'évolution ou du comportement de l'organisme humain ou animal, par exemple au niveau du contrôle de la fécondité d'une espèce animale ou de l'orientation dirigée du métabolisme de l'organisme de l'hôte humain ou animal vers la suppression de certaines hormones, conjointement avec un ou plusieurs effets de vaccination contre un ou plusieurs antigènes distincts.

Dans le domaine vétérinaire, on citera aussi la production accrue de certains constituants, au détriment d'autres constituants, ou la castration immunologique dans le but d'accroître chez l'animal la production de viande.

L'invention a donc encore pour objet des polyvaccins dans lesquels l'une des séquences d'haptène est constituée soit par une hormone, soit par une séquence peptidique ayant une partie commune avec l'hormone, l'hormone appartenant à la classe des hormones peptidiques ou glycopeptidiques. Une hormone particulièrement intéressante à cet égard est constituée par le facteur de libération de l'hormone lutéostimulante LH-RH.

L'invention concerne également encore des réactifs biologiques essentiellement formés à partir de ces polyvaccins pour former in vivo des "cocktails" d'anticorps. Ces anticorps peuvent à leur tour être utiles pour la constitution de réactifs permettant par exemple l'étude de l'action de médicaments à l'étude à l'égard d'un hôte vivant. En particulier, ces anticorps peuvent permettre la détection de récepteurs cellulaires, ou encore l'étude du métabolisme et du mode d'action des médicaments en cause. L'invention concerne donc encore, et ce d'une façon générale, les anticorps eux-mêmes et leur préparation, notamment par administration à un hôte vivant des polyvaccins selon l'invention.

Les revendications s'étendent naturellement également à tous équivalents des molécules synnthétiques qui ont été particulièrement définies. Parmi ces équivalents entrent par exemple des molécules synthétiques polypeptidiques modifiées sans pour autant perdre leurs propriétés immunogènes caractéristiques. Parmi ces équivalents on mentionne les molécules synthétiques polypeptidiques comportant encore un ou plusieurs groupements muramylpeptide greffés sur leur chaîne polypeptidique.

De même, des molécules synthétiques contenant des séquences peptidiques supplémentaires s'ajoutant aux différents haptènes qui les constituent doivent être considérées comme des équivalents des molécules synthétiques plus particulièrement revendiquées, surtout lorsque ces séquences supplémentaires ne modifient pas l'immunogénicité de l'ensemble ou sont elles-mêmes immunogéniquement inertes.

34

<u>REVENDICATIONS</u>

1 - Molécule synthétique caractérisée par la réunion dans une seule molécule d'une pluralité d'haptènes distincts, de préférence de nature peptidique, conjugués deux à deux ou les uns à d'autres par l'intermédiaire de liaisons covalentes, cette molécule synthétique étant exempte de toute chaîne ou groupe porteur ayant un poids moléculaire plus élevé que celui de chacun des susdits haptènes distincts, ces haptènes distincts étant respectivement porteurs d'épitopes ou de sites antigéniques également distincts, chacun de ces haptènes correspondant à un antigène ou à une autre molécule contre laquelle une vaccination ou l'induction d'anticorps est recherchée.

2 - Molécule synthétique selon la revendication 1, caractérisée en ce qu'elle contient de 2 à 10, et notamment de 4 à 6, séquences hapténiques portant des épitopes distincts.

3 - Molécule synthétique selon la revendication 2, caractérisée par la présence d'éléments de liaisons entre haptènes, dont la proportion relative exprimée en proportion de poids moléculaire ne dépasse pas 30 % du poids moléculaire total moyen de ladite molécule.

4 - Molécule synthétique suivant la revendication 3, caractérisée en ce que les éléments de liaisons ont des poids moléculaires respectifs inférieurs à 500, de préférence à 200.

5 - Molécule synthétique selon l'une quelconque des revendications 1 à 4, caractérisée en ce que chacun des haptènes a un poids moléculaire ne dépassant pas environ 5000, de préférence inférieur à 3000.

6 - Molécule synthétique selon l'une quelconque des revendications 1 à 5, caractérisée en ce qu'elle a un poids moléculaire total se situant entre environ 6000 et environ 60.000, de préférence entre environ 10.000 et environ 40.000.

7 - Molécule synthétique selon l'une quelconque des revendications 1 à 6, caractérisée en ce que l'un des haptènes entrant dans la constitution de ladite molécule est dérivé de la protéine M de la surface de Streptococcus pyogenes.

8 - Molécule synthétique selon la revendication 7, caractérisée en ce qu'elle contient des haptènes comportant respectivement des épitopes de la toxine diphtérique, de l'antigène de surface de virus de l'hépatite B et d'un antigène protecteur contre la malaria.

9 - Molécule synthétique caractérisée par le fait que les divers haptènes distincts qu'elle contient sont originaires de la même catégorie d'antigènes, par exemple de sérotypes différents.

10 - Molécule synthétique caractérisée par le fait que les divers haptènes entrant dans sa constitution correspondent à des séquences protéiques distinctes soit d'un même type de poliovirus, soit de poliovirus de types différents.

11 - Composition immunogène administrable "in vivo" et capable d'induire la production d'anticorps actifs distincts reconnaissant des épitopes différents entre eux, caractérisée par l'association avec un véhicule pharmaceutiquement acceptable approprié à l'administration à un hôte vivant de la molécule synthétique, conforme à l'une quelconque des revendications 1 à 10, dans laquelle les haptènes distincts sont respectivement porteurs des susdits épitopes distincts.

12 - Composition selon la revendication 11, caractérisée en ce que chacun des haptènes porte un épitope inducteur d'anticorps protecteurs contre un agent pathogène porteur d'une séquence correspondant à cet épitope.

13 - Composition selon la revendication 11, ou la revendication 12, formulée en doses unitaires de 0,1 à 1 mg de ladite molécule synthétique.

14 - Composition selon l'une quelconque des revendications 11 à 13, conditionnée sous forme de préparation administrable notamment par la voie parentérale ou par la voie orale.

15 - Composition selon l'une quelconque des revendications 11 à 14 également caractérisée par l'association avec un muramylpeptide, celui-ci devant être administré simultanément avec ladite molécule ou de façon décalée, dans des conditions permettant la manifestation de l'activité adjuvante immunologique dudit muramylpeptide vis-à-vis de la susdite molécule synthétique.

16 - Composition selon la revendication 13, caractérisée en ce que le muramylpeptide est constitué par le murabutide.

Office européen
des brevets

**RAPPORT DE RECHERCHE EUROPEENNE**

0180490

Numéro de la demande

EP   85 40 1772

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,D | BIOLOGICAL ABSTRACTS, vol. 78, no. 3, 1984, page 2207, résumé no. 19453; Philadelphia, US; L. CHEDID et al.: "Antibody responses elicited by a polyvalent vaccine containing synthetic diphtheric, streptococcal and hepatitis peptides coupled to the same carrier", & BIOCHEM BIOPHYS RES COMMUN 117(3): 908-915, 1983 * Résumé * | 1-14 | A 61 K   39/385 A 61 K   39/295 |
| Y,D | EP-A-0 003 833  (CIBA-GEIGY AG.) * Page 1, ligne 7 - page 11, ligne 13 * | 15,16 | |
| Y,D | EP-A-0 089 290  (ANVAR) * Page 3, ligne 19 - page 4, ligne 35 * | 15,16 | **DOMAINES TECHNIQUES RECHERCHES (Int. Cl.4)** |
| A,D | FR-A-2 428 050  (AGENCE NATIONALE DE VALORISATION DE LA RECHERCHE) | | A 61 K |

Le présent rapport de recherche a été établi pour toutes les revendications

| Lieu de la recherche | Date d'achèvement de la recherche | Examinateur |
|---|---|---|
| LA HAYE | 18-12-1985 | REMPP G.L.E. |